# EUROPEAN PATENT SPECIFICATION

(11) **EP 3 382 395 B1**
(45) Date of publication and mention of the grant of the patent: **21.04.2021**
(21) Application number: 16868687.1
(22) Date of filing: 25.11.2016
(51) Int. Cl.: G01N 33/569, G01N 33/53, G01N 33/543, C07K 16/08, C12N 15/09

(54) **IMMUNOCHROMATOGRAPHIC ANALYSIS DEVICE, METHOD AND KIT FOR DETECTING VARICELLA ZOSTER VIRUS**
IMMUNOCHROMATOGRAPHISCHE VORRICHTUNG, METHODE UND KIT ZUR DETEKTION VON VARIZELLA ZOSTER VIRUS
APPAREIL ET MÉTHODE IMMUNOCHROMATOGRAPHIQUE AINSI QU'UN KIT POUR DÉTECTER LE VIRUS VARICELLE-ZONA

(30) Priority: 26.11.2015 JP 2015230928
(43) Date of publication of application: 03.10.2018
(73) Proprietor: Tanaka Kikinzoku Kogyo K.K., Chiyoda-ku Tokyo 100-6422 (JP)
(72) Inventor: SUZUKI Keita, Hiratsuka-shi Kanagawa 254-0076 (JP); IWAMOTO Hisahiko, Hiratsuka-shi Kanagawa 254-0076 (JP); MOCHIZUKI Hiroko, Hiratsuka-shi Kanagawa 254-0076 (JP)
(74) Representative: Hoffmann Eitle
(86) International application number: PCT/JP2016/085018
(87) International publication number: WO 2017/090744

(56) References cited:
- JP-A- 2010 019 786
- JP-A- 2010 019 786
- FOWLER W J ET AL: "IDENTIFICATION OF IMMUNODOMINANT REGIONS AND LINEAR BETA CELL EPITOPES OF THE GEPSILON PROTEIN OF VARICELLA-ZOSTER VIRUS", VIROLOGY, ELSEVIER, AMSTERDAM, NL, vol. 214, no. 2, 1 January 1995 (1995-01-01), pages 531-540, XP000919259, ISSN: 0042-6822, DOI: 10.1006/VIRO.1995.0064
- CUZZUBBO ANDREA J ET AL: "Use of recombinant envelope proteins for serological diagnosis of dengue virus infection in an immunochromatographic assay", CLINICAL AND DIAGNOSTIC LABORATORY IMMUNOLOGY, AMERICAN SOCIETY FOR MICROBIOLOGY, US, vol. 8, no. 6, 1 November 2001 (2001-11-01), pages 1150-1155, XP002312032, ISSN: 1071-412X
- E. I. LADERMAN ET AL: "Rapid, Sensitive, and Specific Lateral-Flow Immunochromatographic Point-of-Care Device for Detection of Herpes Simplex Virus Type 2-Specific Immunoglobulin G Antibodies in Serum and Whole Blood", CLINICAL AND VACCINE IMMUNOLOGY, vol. 15, no. 1, 14 November 2007 (2007-11-14), pages 159-163, XP055184798, ISSN: 1556-6811, DOI: 10.1128/CVI.00218-07
- FOWLER WJ ET AL.: 'Identification of Immunodominant Regions and Linear B Cell Epitopes of the gE Envelope Protein of Varicella-Zoster Virus' VIROLOGY vol. 214, 1995, pages 531 - 540, XP000919259

## Description

### Technical Field

The present invention relates to an immunochromatographic analysis device for detecting Varicella-Zoster Virus, an immunochromatographic analysis kit, and a detection method therefor.

### Background Art

Varicella-Zoster Virus (VZV) is a virus which belongs to the α-herpesvirinae and has a linear double-stranded DNA composed of about 125 kbp base sequence.

This virus causes varicella in the initial infection in humans and remains dormant in satellite cells around nerve cells in an inactive stage after healing. When immunocompetence is reduced due to some causes, the dormant virus is reactivated to cause zoster. When a human in an immunosuppressed state (who uses an immunosuppressive agent, or who has an underlying disease such as malignant tumor or immunodeficiency) is initially infected with VZV or is infected with reactivated VZV, severe conditions may occur, and sometimes deadly conditions may occur. Therefore, establishment of a rapid diagnostic method for performing an early treatment has been awaited.

Patent Literature 1 discloses a preventive or therapeutic agent for a neurological disease using an antibody which recognizes a Varicella-Zoster Virus immediate-early protein IE62 and also crossreacts with a brain-derived neurotrophic factor as a means for prevention or treatment of nerve pain or the like after zoster.

Patent Literature 2 discloses an inhibitor of endocytosis-dependent DNA uptake which enhances the function of a DNase X protein, and discloses a method for indirectly predicting a pathological condition caused by Varicella-Zoster Virus or the like using human DNase X as an antigen.

Patent Literature 3 discloses immunochromatographic devices that involve the use of a non-ionic surfactants.

Non-Patent Literature 1 aims to explore the immunodominant regions of the gE envelope protein of Varicella-Zoster Virus in order to elucidate regions which would be suitable for developing a low cost, safe, and potent subunit vaccine.

Non-Patent Literature 2 discloses an immunochromatographic device that detects immunoglobulins that bind to viral envelope glycoproteins of dengue viruses 1, 2, 3, and 4.

Non-Patent Literature 3 discloses the development and evaluation of a Herpes simplex virus type 2 immunoglobulin G-specific antibody lateral-flow immunochromatographic assay (LFIA) based on colloidal gold nanoparticles.

Non-Patent Literature 4 discloses the evaluation of laboratory methods for diagnosis of varicella including an indirect ELISA assay, a direct fluorescent antibody assay and a PCR-based assay.

### Background Art Literature

### Patent Literature

Patent Literature 1: Japanese Patent No. 5279504
Patent Literature 2: JP-A-2008-253188
Patent Literature 3: JP 2010 019786 A

### Non-Patent Literature

Non-Patent Literature 1: Fowler et al., 1995. Virology. 214(2):531-40
Non-Patent Literature 2: Cuzzubbo et al., 2001. Clin Diagn Lab Immunol. 8(6):1150-5
Non-Patent Literature 3: Laderman et al., 2008. Clin Vaccine Immunol. 15(1):159-63
Non-Patent Literature 4: Leung et al., 2010. Clin Infect Dis. 51(1):23-32

### Summary of Invention

### Problems to be Solved by Invention

However, the method described in Patent Literature 1 is a method for detecting the cause of nerve pain induced by VZV or treating nerve pain, and is not a method for specifically detecting VZV. Further, the method described in Patent Literature 2 is also not a method for directly detecting VZV itself, but is a method for indirectly predicting a pathological condition using human DNase X as an antigen.

In this manner, a method for specifically detecting VZV has not been established so far, and infection with VZV could not be diagnosed rapidly. Therefore, an object of the present invention is to provide a means for diagnosing infection with VZV rapidly.

### Means for Solving Problems

VZV has Varicella-Zoster Virus glycoprotein E (hereinafter also referred to as "VZVgE") which is a glycoprotein and has a function to capture Fc region in an immunoglobulin G This VZVgE has an about 50% homology in the amino acid sequence with glycoprotein E (gE) of the other α-herpesviruses (for example, HSV, BHV, FeHV, *etc*.), and the gE proteins of these viruses have similar functions and structures.

The inventors of the present invention conducted intensive studies by focusing on the above-mentioned VZVgE as a means for directly detecting VZV, and as a result, they found that an antibody which recognizes a certain specific region of VZVgE can specifically detect VZV without showing crossreactivity with the other α-herpesviruses.

Then, they found that infection of humans with VZV can be diagnosed rapidly and simply by using an immunochromatographic analysis device using an antibody which recognizes a specific region of VZVgE, and thus accomplished the present invention.

Namely, the present invention is described below.
1. An immunochromatographic analysis device, which is an immunochromatographic analysis device for detecting Varicella-Zoster Virus (VZV), containing a sample addition part, a labeling substance retaining part, a chromatographic medium part having a detection part, and an absorption part,
   wherein at least one of the labeling substance retaining part and the detection part contains an antibody which recognizes the amino acid sequence at positions 1 to 188 of glycoprotein E of the Varicella-Zoster Virus (VZVgE) composed of the amino acid sequence of SEQ ID NO: 1.
2. An immunochromatographic analysis device, which is an immunochromatographic analysis device for detecting Varicella-Zoster Virus (VZV), containing a sample addition part, a labeling substance retaining part, a chromatographic medium part having a detection part, and an absorption part,
   wherein at least one of the labeling substance retaining part and the detection part contains an antibody which recognizes the amino acid sequence at positions 108 to 135 of glycoprotein E of the Varicella-Zoster Virus (VZVgE) composed of the amino acid sequence of SEQ ID NO: 1.
3. The immunochromatographic analysis device according to item 2, wherein at least one of the labeling substance retaining part and the detection part comprises an antibody which recognizes the amino acid sequence at positions 108 to 135 of glycoprotein E of the varicella-zoster virus (VZV gE) composed of the amino acid sequence of SEQ ID NO: 1, and the other part comprises an antibody which recognizes the amino acid sequence at positions 48 to 88 of glycoprotein E of the varicella-zoster virus (VZV gE) composed of the amino acid sequence of SEQ ID NO: 1.
4. The immunochromatographic analysis device according to any one of items 1 to 3, wherein a sample to be added to the sample addition part is a blister's fluid containing the Varicella-Zoster Virus.
5. The immunochromatographic analysis device according to any one of items 1 to 4, wherein a labeling substance contained in the labeling substance retaining part is a gold nanoparticle, and the labeling substance retaining part contains the gold nanoparticles in an amount of 0.05 to 1.5 µg/cm².
6. An immunochromatographic analysis kit containing the immunochromatographic analysis device according to any one of items 1 to 5, and a specimen diluent for diluting and developing a specimen.
7. The immunochromatographic analysis kit according to item 6, wherein the specimen diluent contains at least one type of nonionic surfactant.
8. An immunochromatographic analysis method, which is a method for detecting Varicella-Zoster Virus (VZV) in a specimen using an immunochromatographic analysis device including a sample addition part, a labeling substance retaining part, a chromatographic medium part having a detection part, and an absorption part, wherein at least one of the labeling substance retaining part and the detection part of the immunochromatographic analysis device contains an antibody which recognizes the amino acid sequence at positions 1 to 188 of glycoprotein E of the Varicella-Zoster Virus (VZVgE) composed of the amino acid sequence of SEQ ID NO: 1, and the method includes the following steps (1) to (4):
   (1) a step of adding solution containing specimen obtained by diluting a specimen with a specimen diluent to the sample addition part;
   (2) a step of allowing an antibody retained in the labeling substance retaining part to recognize Varicella-Zoster Virus;
   (3) a step of developing the specimen and the antibody in the chromatographic medium part as a mobile phase; and
   (4) a step of detecting Varicella-Zoster Virus in the developed mobile phase by an antibody contained in the detection part.

### Effects of Invention

The present invention is an immunochromatographic analysis device using an antibody which binds to a specific region of glycoprotein E (gE) of Varicella-Zoster Virus (VZV). By using the immunochromatographic analysis device of the present invention, VZV can be specifically detected rapidly and simply. Namely, the diagnosis of varicella or zoster can be performed more reliably and rapidly.

### Brief Description of Drawings

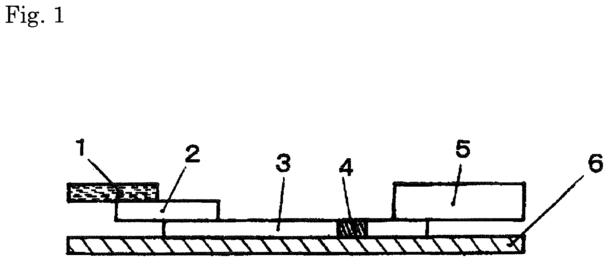

[FIG. 1] FIG. 1 is a cross-sectional view for illustrating the structure of an immunochromatographic analysis device of one embodiment of the present invention.

### Description of Embodiments

Hereinafter, embodiments for carrying out the present invention will be described.

The immunochromatographic analysis device for detecting Varicella-Zoster Virus (VZV) of the present invention includes a sample addition part where a specimen is added, a labeling substance retaining part where a labeling substance is retained, a chromatographic medium part having a detection part where VZV is detected, and an absorption part where a liquid having passed through the detection part is absorbed, and is characterized in that both the labeling substance retaining part and the detection part contain an antibody which recognizes VZV glycoprotein E (hereinafter also referred to as "VZVgE"), and in particular, at least one of the labeling substance retaining part and the detection part contains an antibody which recognizes the amino acid sequence at positions 1 to 188 of VZVgE composed of the amino acid sequence of SEQ ID NO: 1.

Preferably, at least one of the labeling substance retaining part and the detection part contains an antibody which recognizes the amino acid sequence at positions 48 to 135 of VZVgE composed of the amino acid sequence of SEQ ID NO: 1.

More preferably, at least one of the labeling substance retaining part and the detection part contains an antibody which recognizes at least one of the amino acid sequences at positions 48 to 88, at positions 89 to 107, and at positions 108 to 135 of VZVgE composed of the amino acid sequence of SEQ ID NO: 1.

Particularly preferably, at least one of the labeling substance retaining part and the detection part contains an antibody which recognizes the amino acid sequence at positions 108 to 135 of VZVgE composed of the amino acid sequence of SEQ ID NO: 1.

Hereinafter, a case where at least one of the labeling substance retaining part and the detection part contains an antibody which recognizes the amino acid sequence at positions 1 to 188 of VZVgE composed of the amino acid sequence of SEQ ID NO: 1 will be described as an example, however, a preferred embodiment of the antibody contained in at least one of the labeling substance retaining part and the detection part is as described above.

Varicella-Zoster Virus (VZV) belongs to the α-herpesvirinae and is a pathogenic mediator of varicella and zoster. Varicella-Zoster Virus glycoprotein E (VZVgE) which is one of the glycoproteins of this VZV is composed of 623 amino acids represented by SEQ ID NO: 1, and is constituted by an extracellular domain corresponding to the amino acid sequence at positions 1 to 544, a transmembrane domain (TM) corresponding to the amino acid sequence at positions 545 to 562, and a cytoplasmic tail corresponding to the amino acid sequence at positions 563 to 623 (Jurie K. et al., Journal of Virology, Jan. 1997, pp. 110-119).

Among the antibodies to be used in the present invention, an antibody contained in at least one of the labeling substance retaining part and the detection part of the below-mentioned immunochromatographic analysis device recognizes the amino acids at positions 1 to 188 of the amino acid sequence of VZVgE. Here, in this description, the word "recognizes" in the phrase "an antibody recognizes a certain specific amino acid sequence" means that the antibody binds to the specific amino acid sequence with higher affinity than the other amino acid sequences.

As described above, VZVgE has an about 50% amino acid sequence homology with the gE of the other α-herpesviruses. In the present invention, by using an antibody which recognizes the amino acid sequence at positions 1 to 188 of VZVgE, an immunochromatographic analysis device which specifically detects VZV without crossreaction with the other α-herpesviruses with a high homology can be provided.

In the immunochromatographic analysis device of the present invention, an antibody which recognizes the amino acid sequence at positions 1 to 188 of VZVgE is previously incorporated in at least one of the labeling substance retaining part and the detection part thereof. Namely, the above-mentioned antibody may be previously incorporated only in the labeling substance retaining part, or the above-mentioned antibody may be previously incorporated only in the detection part, or the above-mentioned antibody may be previously incorporated in both the labeling substance retaining part and the detection part.

Above all, it is preferred that the above-mentioned antibody may be previously incorporated in only either one of the labeling substance retaining part and the detection part. This is considered to be because when a region of VZVgE recognized by an antibody (hereinafter also referred to as "first antibody") supported on the labeling substance retaining part and a region of VZVgE recognized by an antibody (hereinafter also referred to as "second antibody") supported on the detection part are different from each other, a sandwich structure as shown below is formed without masking the epitope by antibody binding, or without undergoing an interaction such as an effect of the conformational change of an antigen, and therefore, the detection sensitivity is improved.

Namely, firstly, the antibody (first antibody) supported on the labeling substance retaining part binds to a portion of VZVgE. Subsequently, the antibody (second antibody) fixed on the detection part binds to another portion which is different from the portion to which the first antibody binds, whereby a sandwich structure is formed such that VZVgE is interposed between the antibodies, and as a result, it is considered that VZV is detected.

### (Method for Producing Antibody)

In this description, the "antibody" includes natural antibodies such as a polyclonal antibody and a monoclonal antibody, a chimeric antibody, a humanized antibody, and a single-stranded antibody, which can be produced by using a gene recombination technique, a human antibody which can be produced by using a human antibody-producing transgenic animal or the like, an antibody produced by phage display, and binding fragments thereof.

Examples of an animal species which produces the antibody include a human, a mouse, a rat, a rabbit, a goat, and a horse. The immunoglobulin may be any of IgG, IgM, IgA, IgE, and IgD.

In one embodiment, the VZVgE peptide as an immunogen can be produced by a known general production method. Namely, a gE protein extracted and purified from VZV containing the amino acid sequence of SEQ ID NO: 1, or a gE protein obtained by expressing a cloned gE protein gene in a host such as *E. coli* through genetic engineering followed by extraction and purification, or further a polypeptide constituting a portion of the gE protein can be used as an immunogen.

The monoclonal antibody is obtained according to a conventional method as follows. The spleen cells and myeloma cells of a mouse immunized with the above-mentioned immunogen are fused, and a hybridoma which produces a target antibody is selected, and a monoclonal antibody produced from this hybridoma is obtained [see, for example, the method of Kohler and Milstein [Nature, 256 (1975), 495-497].

The polyclonal antibody is obtained by separating a target antibody from an antiserum obtained by immunizing an antibody-producing animal (for example, a human, a mouse, a rat, a rabbit, a goat, a horse, *etc*.) with the above-mentioned immunogen according to a conventional method.

The screening of the hybridoma clone which produces a monoclonal antibody can be performed by culturing the hybridoma in, for example, a microtiter plate, and the reactivity with the immunogen in a culture supernatant in a well in which proliferation is observed is measured by, for example, an enzyme immunoassay method such as ELISA.

This hybridoma is cultured using a culture medium (for example, DMEM containing 10% fetal bovine serum), and the centrifugal supernatant of the culture solution can be used as a monoclonal antibody solution. Further, this hybridoma is injected into the abdominal cavity of an animal from which this hybridoma is derived so as to allow the animal to produce an ascites, and the obtained ascites can be used as a monoclonal antibody solution. The monoclonal antibody is preferably isolated and/or purified.

Among the antibodies which recognize VZVgE, an antibody which recognizes a specific region of the amino acid sequence of VZVgE can be obtained by, for example, selecting a hybridoma which produces an antibody that shows a higher reactivity with the specific region of the amino acid sequence of VZVgE from the hybridomas which produce an antibody that recognizes VZVgE by Western blotting or the like with using a fragment of a protein corresponding to the specific region of the amino acid sequence of VZVgE.

For example, the antibody which specifically recognizes the amino acid sequence at positions 1 to 188 of VZVgE is obtained by performing screening as described below. For example, as described later in Examples, the antibody can be obtained by selecting a hybridoma which produces an antibody that shows a higher reactivity with the amino acid sequence at positions 1 to 188 of VZVgE among the hybridomas which produce the antibody that recognizes VZVgE using a protein fragment at positions 1 to 188 of the amino acid sequence of VZVgE. The antibodies which specifically recognize the amino acid sequences at positions 48 to 88, at positions 89 to 107, and at positions 108 to 135 of VZVgE can also be obtained in the same manner as described above.

Hereinabove, the method for producing the antibody to be used in the present invention has been described, and will be specifically described in detail in Examples.

### (Immunochromatographic Analysis Device of the Present Invention)

Next, one embodiment of the immunochromatographic analysis device of the present invention will be described with reference to the drawing. In this connection, in this description, the "fixing" means that the antibody is placed on a carrier such as a membrane so that the antibody does not move, and the "retaining" means that the antibody is movably placed in a carrier such as a membrane or on the surface thereof.

As shown in FIG. 1, according to one embodiment of the immunochromatographic analysis device of the present invention, the device is constituted by a sample addition part (1), a labeling substance retaining part (2), a chromatographic medium part (3), a detection part (4), an absorption part (5), and a backing sheet (6).

The sample addition part (1) is a part where a sample which contains a specimen is added in the immunochromatographic analysis device. The sample addition part (1) can be constituted by a porous sheet having a property such that the sample is rapidly absorbed and the specimen promptly migrates therein. Examples of the porous sheet include a cellulose filter paper, glass fiber, polyurethane, polyacetate, cellulose acetate, nylon, and a cotton cloth.

The labeling substance retaining part (2) contains the below-mentioned labeling substance, and the labeling substance is retained in the labeling substance retaining part (2) as a labeling antibody in which the labeling substance and an antibody are bound to each other. The antibody (first antibody) which binds to the labeling substance is an antibody which recognizes VZVgE as described above.

In a case where the antibody (second antibody) which is incorporated in the below-mentioned detection part is not an antibody which recognizes the amino acid sequence at positions 1 to 188 of VZVgE among the antibodies which recognize VZVgE, as the antibody (first antibody) which binds to the labeling substance, an antibody which recognizes the amino acid sequence at positions 1 to 188 of VZVgE is used. When a specimen migrates in the labeling substance retaining part, the labeling antibody (first antibody) and VZV in the specimen bind to each other. In the labeling substance retaining part (2), glass fiber and a membrane of cellulose or the like are generally used.

The content of the antibody (first antibody) in the labeling substance retaining part is generally from 0.03 to 0.50 µg/device, preferably from 0.05 to 0.4 µg/device, more preferably from 0.1 to 0.3 µg/device. Further, the content of the antibody (first antibody) per unit area of the labeling substance retaining part is generally from 0.05 to 1.0 µg/cm², preferably from 0.1 to 0.8 µg/cm², and more preferably from 0.17 to 0.6 µg/cm².

In the labeling of a detection reagent in an immunochromatographic analysis, an enzyme or the like is also generally used, however, it is preferred to use an insoluble carrier as the labeling substance since it is suitable for determining the presence of a detection target by visual observation. The labeled detection reagent can be prepared by sensitizing the antibody to the insoluble carrier. In this connection, a method for sensitizing the antibody to the insoluble carrier may be performed in accordance with a known method.

As the insoluble carrier to serve as the labeling substance, metal particles such as gold, silver, or platinum, metal oxide particles such as iron oxide, non-metal particles such as sulfur, latex particles composed of a synthetic polymer, or other insoluble carriers can be used.

As described above, the insoluble carrier is the labeling substance suitable for visually determining the presence of the detection target, and is preferably a colored substance in order to facilitate the determination by visual observation. The metal particles and the metal oxide particles exhibit a specific natural color per se according to the particle diameter, and the color can be utilized as a label.

In particular, gold nanoparticles are preferred since detection is simple, aggregation is less likely to occur, and also nonspecific color development is less likely to occur. The average particle diameter of the gold nanoparticles is, for example, from 10 nm to 250 nm, and preferably from 35 nm to 120 nm. The average particle diameter can be obtained by randomly measuring the projected area circle equivalent diameters of 100 particles using a projected image taken by a transmission electron microscope (TEM, manufactured by JEOL Ltd., JEM-2010), and calculating the average of the projected area circle equivalent diameters.

The amount of the gold nanoparticles contained in the labeling substance retaining part per unit area of the labeling substance retaining part is generally from 0.025 to 1.5 µg/cm², preferably from 0.05 to 1.5 µg/cm², more preferably from 0.1 to 1.0 µg/cm ², further more preferably from 0.2 to 0.6 µg/cm². This is because by setting the amount within the above range, the labeled particles are developed with being dispersed, and high sensitivity can be achieved without inhibiting the recognition site of the antibody.

The chromatographic medium part (3) is a developing part in a chromatograph. The chromatographic medium part (3) is an inactive membrane composed of a microporous material exhibiting a capillary phenomenon. From the viewpoint of having no reactivity with a detection reagent, a fixing reagent, a detection target, and the like to be used in the chromatograph and also from the viewpoint of enhancing the effect of the present invention, for example, a membrane made of nitrocellulose (hereinafter also referred to as "nitrocellulose membrane") or a membrane made of cellulose acetate (hereinafter also referred to as "cellulose acetate membrane") is preferred, and a nitrocellulose membrane is more preferred. In this connection, it is also possible to use a cellulose-based membrane, a nylon membrane, and a porous plastic cloth (polyethylene, polypropylene, *etc*.).

The nitrocellulose membrane may be any as long as it contains nitrocellulose as a main component, and a membrane which contains nitrocellulose as a main material such as a pure product or a nitrocellulose mixed product can be used.

It is also possible to further incorporate a substance which promotes a capillary phenomenon in the nitrocellulose membrane. As the substance, a substance which lowers the surface tension of the membrane surface to impart hydrophilicity is preferred. For example, a substance which has an amphiphilic action; does not affect the migration of the detection target; and does not affect the color development of the labeling substance such as a saccharide, an amino acid derivative, a fatty acid ester, any of a variety of synthetic surfactants, or an alcohol is preferred.

The nitrocellulose membrane is a porous material and exhibits a capillary phenomenon. The index of this capillary phenomenon can be confirmed by measuring a water absorption speed (a water absorption time: a capillary flow time). The water absorption speed affects the detection sensitivity and the test time.

The form and size of the chromatographic medium part (3) represented by a nitrocellulose membrane or a cellulose acetate membrane as described above are not particularly limited, and may be any as long as they are appropriate in terms of actual operation and observation of the reaction result.

Further, in order to make the operation simpler, it is preferred to provide a support made of a plastic or the like on the rear surface of the chromatographic medium part (3). The property of this support is not particularly limited, however, in a case where the measurement result is observed by visual determination, the support preferably has a color which is not similar to the color brought about by the labeling substance, and is generally preferably colorless or white.

The detection part (4) is formed on the chromatographic medium part (3). Namely, an antibody which recognizes VZV is fixed at an arbitrary position on the chromatographic medium part (3). The fixing of the antibody can be performed according to a conventional method. As for the antibody (second antibody), in a case where the antibody (first antibody) which binds to the labeling substance is not an antibody which recognizes the amino acid sequence at positions 1 to 188 of VZVgE among the antibodies which recognize VZVgE, as the antibody (second antibody) which is incorporated in the detection part, an antibody which recognizes the amino acid sequence at positions 1 to 188 of VZVgE is used.

The content of the antibody (second antibody) in the detection part (4) is generally from 0.1 to 3.0 µg/device, preferably from 0.3 to 2.0 µg/device, and more preferably from 0.3 to 1.0 µg/device. Further, the content of the antibody (second antibody) per unit area of the detection part (4) is generally from 0.04 to 1.0 µg/cm², preferably from 0.125 to 0.8 µg/cm², and more preferably from 0.125 to 0.42 µg/cm².

Further, on the chromatographic medium part (3), in order to prevent the decrease in the analysis accuracy due to nonspecific adsorption, according to need, the chromatographic medium part (3) may be subjected to a blocking treatment by a known method. In general, in the blocking treatment, a protein such as bovine serum albumin, skim milk, casein, or gelatin is preferably used. After such a blocking treatment, according to need, for example, washing may be performed by using one surfactant or a combination of two or more surfactants selected from Tween 20, Triton X-100, SDS, *etc.*

In the detection part (4), an anti-IgG antibody application part (control region) on which an anti-IgG antibody is fixed as a control other than the above-mentioned second antibody may be provided. The region is configured such that the labeling substance of the labeling antibody which did not react with the antigen or the labeling substance of the labeling antibody which did not react with the second antibody reacts with the anti-IgG antibody in this anti-IgG antibody application part and is fixed thereon, and therefore functions as a control which shows that development is carried out normally.

The absorption part (5) is provided for absorbing a liquid of the specimen, the developing solution, or the like having passed through the detection part (4) at an end of the chromatographic medium part (3). In the immunochromatographic analysis device of the present invention, as the absorption part (5), for example, a material in which a polymer such as an acrylic acid polymer and a hydrophilic agent having an ethylene oxide group or the like are incorporated in glass fiber, pulp, cellulose fiber, or the like or a non-woven fabric thereof is used. Particularly preferably, glass fiber is used. When the absorption part (5) is composed of glass fiber, the backward migration of the sample solution can be greatly reduced.

The backing sheet (6) is a base material. By applying an adhesive to one surface or sticking an adhesive tape to one surface, the surface has adhesiveness, and on the adhesive surface, part or all of the sample addition part (1), the labeling substance retaining part (2), the chromatographic medium part (3), the detection part (4), and the absorption part (5) are provided in close contact with the surface. The backing sheet (6) is not particularly limited as the base material as long as it becomes impermeable to a sample solution and also is impermeable to moisture by the adhesive.

The immunochromatographic analysis device prepared as described above is generally subjected to a drying treatment before making into a product. The drying temperature is, for example, from 20 to 50°C, and the drying time is from 0.5 to 1 hour.

### (Immunochromatographic Analysis Kit of the Present Invention)

The immunochromatographic analysis kit of the present invention includes the above-mentioned immunochromatographic analysis device and a specimen diluent for diluting and developing a specimen.

The specimen diluent in the immunochromatographic analysis kit of the present invention can also be used as a developing solution. In general, water is used as a solvent, and a buffer solution, a salt, and a nonionic surfactant, and further, for example, one type or two or more types of a protein, a polymeric compound (such as PVP), a nonionic surfactant or a polyanion, or an antimicrobial agent, a chelating agent, *etc.* for promoting an antigen-antibody reaction or suppressing a nonspecific reaction may be added thereto.

Examples of the nonionic surfactant include Triton X-100 (trade name, polyethylene glycol mono-p-isooctylphenyl ether), Tween 20 (trade name, polyoxyethylene sorbitan monolaurate), NP-40 (trade name, Nonidet 40), Brij 35, and NONION MN-811 (manufactured by NOF CORPORATION), and one type or two or more types may be added.

Preferably, the nonionic surfactant contained in the specimen diluent includes one or more types of nonionic surfactants having an HLB value of 6 to 12. More preferably, it includes one or more types of nonionic surfactants having an HLB value of 7 to 11, and most suitably having an HLB value of 8 to 10.

As the nonionic surfactant having an HLB value of 6 to 12, preferably, a polyoxyalkylene ether with a hydroxy group at one end is used. An alkyl group at one end may be linear or branched, and also an alkyl moiety of a repeating alkyl ether group may be linear or branched.

Further, it is more preferred that the nonionic surfactant contained in the specimen diluent not only includes one or more types of nonionic surfactants having an HLB value of 6 to 12, but also includes one or more types of nonionic surfactants having an HLB value of 13 to 18.

As for the blending ratio thereof, the mass ratio of the total amount (B) of the nonionic surfactants having an HLB value of 13 to 18 to the total amount (A) of the nonionic surfactants having an HLB value of 6 to 12 is preferably as follows: A:B = 30:70 to 70:30. More preferably, these nonionic surfactants are used at a blending ratio as follows: A:B = 40:60 to 60:40.

Alternatively, in order to extract the detection target (VZVgE or the like) from the specimen in the specimen diluent, a known hydrophobic substance such as an alcohol may be incorporated in the specimen diluent or may be added thereto immediately after collecting the specimen. By incorporating or adding the hydrophobic substance in the specimen diluent, the detection target (VZVgE or the like) can be extracted from the specimen at the same time as when the specimen is diluted, and therefore, the time and labor for extraction in advance can be omitted, and also the detection sensitivity can be enhanced, and thus, this operation is preferred.

In a case where the specimen diluent is used as a developing solution, solution containing specimen obtained by mixing the specimen and the developing solution in advance can be supplied and added onto the sample addition part to effect development, or the specimen is supplied and added onto the sample addition part in advance, and thereafter, the developing solution may be supplied and added onto the sample addition part to effect development.

### (Immunochromatographic Analysis Method of the Present Invention)

The immunochromatographic analysis method of the present invention includes the following steps (1) to (4), and is a method for detecting Varicella-Zoster Virus (VZV) which is a detection target contained in a specimen using the above-mentioned immunochromatographic analysis device, and is an immunochromatographic analysis method, in which at least one of the labeling substance retaining part and the detection part of the immunochromatographic analysis device contains an antibody that recognizes the amino acid sequence at positions 1 to 188 of Varicella-Zoster Virus glycoprotein E (VZVgE) composed of the amino acid sequence of SEQ ID NO: 1, and which includes the following steps (1) to (4).
(1) a step of adding solution containing specimen obtained by diluting a specimen with a specimen diluent to the sample addition part
(2) a step of allowing an antibody retained in the labeling substance retaining part to recognize Varicella-Zoster Virus
(3) a step of developing the specimen and the antibody in the chromatographic medium part as a mobile phase
(4) a step of detecting Varicella-Zoster Virus in the developed mobile phase by an antibody contained in the detection part

Both the labeling substance retaining part and the detection part in the above-mentioned immunochromatographic analysis device used in the immunochromatographic analysis method of the present invention contain an antibody which recognizes VZVgE. In particular, at least one of the labeling substance retaining part and the detection part contains an antibody which recognizes the amino acid sequence at positions 1 to 188 of VZVgE composed of the amino acid sequence of SEQ ID NO: 1.

Further, the antibody contained in at least one of the labeling substance retaining part and the detection part is preferably an antibody which recognizes the amino acid sequence at positions 48 to 135 of VZVgE composed of the amino acid sequence of SEQ ID NO: 1.

More preferably, at least one of the labeling substance retaining part and the detection part contains an antibody which recognizes at least one of the amino acid sequences at positions 48 to 88, at positions 89 to 107, and at positions 108 to 135 of VZVgE composed of the amino acid sequence of SEQ ID NO: 1.

Particularly preferably, at least one of the labeling substance retaining part and the detection part contains an antibody which recognizes the amino acid sequence at positions 108 to 135 of VZVgE composed of the amino acid sequence of SEQ ID NO: 1.

The respective steps will be described below.

### (1) Step of adding specimen-containing solution obtained by diluting specimen with specimen diluent to sample addition part

In the step (1), in the first place, solution containing specimen is preferably formed by appropriately preparing or diluting a specimen with a specimen diluent to such a concentration that the specimen migrates smoothly in the immunochromatographic medium without decreasing the measurement accuracy. As the specimen diluent, the above-mentioned specimen diluent can be used. In the second place, the specimen-containing solution is added onto the sample addition part (1) in a predetermined amount (generally from 0.1 to 2 mL). When the specimen-containing solution is added, the specimen-containing solution starts to migrate in the sample addition part (1).

The specimen used in the present invention is a specimen which may contain Varicella-Zoster Virus which is a detection target. Specifically, although a blister's fluid obtained by puncturing a blister of a patient infected with Varicella-Zoster Virus, a pharyngeal swab, or the like is exemplified, the specimen is not limited thereto.

### (2) Step of allowing antibody retained in labeling substance retaining part to recognize Varicella-Zoster Virus

The step (2) is a step in which the specimen-containing solution added to the sample addition part in the step (1) is allowed to migrate to the labeling substance retaining part (2), and the antibody (first antibody) to which a labeling substance retained in the labeling substance retaining part has bound is allowed to recognize Varicella-Zoster Virus glycoprotein E (VZVgE) which is the detection target in the specimen.

As the labeling substance, the above-mentioned substance can be used. The antibody (first antibody) which binds to the labeling substance is an antibody which recognizes VZVgE as described above. This antibody recognizes and binds to VZVgE in the specimen developed from the sample addition part. Particularly, in a case where the antibody (first antibody) which binds to the labeling substance is the former antibody which recognizes the amino acid sequence at positions 1 to 188 of VZVgE, the antibody recognizes and binds to the amino acid sequence at positions 1 to 188 of VZVgE in the specimen developed from the sample addition part.

### (3) Step of developing specimen and antibody in chromatographic medium part as mobile phase

The step (3) is a step in which after Varicella-Zoster Virus which is the detection target is recognized by the antibody to which the labeling substance has bound in the labeling substance retaining part in the step (2), the specimen and the antibody are allowed to pass on the chromatographic medium part as a mobile phase.

### (4) Step of detecting Varicella-Zoster Virus in developed mobile phase by antibody contained in detection part

The step (4) is a step in which VZVgE in Varicella-Zoster Virus in the specimen having passed on the chromatographic medium part as the mobile phase is specifically reacted and bound so as to be interposed like a sandwich between the antibody fixed on the detection part and the antibody to which the labeling substance has bound in the step (2) by an antigen-antibody specific binding reaction, and the detection part is colored.

The antibody (second antibody) is an antibody which recognizes VZVgE as described above. In a case where the antibody (first antibody) which binds to the labeling substance is the latter antibody which is not an antibody that recognizes the amino acid sequence at positions 1 to 188 of VZVgE among the antibodies which recognize VZVgE, an antibody which recognizes the amino acid sequence at positions 1 to 188 of VZVgE is incorporated in this detection part.

The antibody recognizes and binds to VZVgE in the specimen developed from the labeling substance retaining part. Particularly, in a case where the antibody (second antibody) is the former antibody which recognizes the amino acid sequence at positions 1 to 188 of VZVgE, the antibody recognizes and binds to the amino acid sequence at positions 1 to 188 of VZVgE, which has been developed from the labeling substance retaining part, and to which the first antibody has bound so as to effect capture.

In a case where only either one of the labeling substance retaining part and the detection part contains an antibody which recognizes and binds to the amino acid sequence at positions 1 to 188 of VZVgE, the antibody (second antibody) fixed on the detection part binds to another portion which is different from the portion to which the antibody (first antibody) supported on the labeling substance retaining part has bound so as to interpose the detection target therebetween like a sandwich, and the detection part is colored.

In a case where Varicella-Zoster Virus which is the detection target is not present, the labeling reagent dissolved in an aqueous component of the sample does not cause a specific binding reaction even if it passes through the detection part on the chromatographic medium part, and therefore, the detection part is not colored.

Finally, the aqueous component of the specimen-containing solution migrates to the absorption part (5).

### Examples

Hereinafter, the present invention will be further described by way of Examples, however, the present invention is not limited to the following examples.

### [Test Example 1] Preparation of Antibody which Recognizes VZVgE

The amino acid sequence (SEQ ID NO: 1) of the gE protein of VZV (VZVgE) was obtained from the DDBJ (the data base of the National Institute of Genetics). From the amino acid sequence of VZVgE, an amino acid sequence (at positions 1 to 544) represented by SEQ ID NO: 2, which is an extracellular domain excluding the transmembrane domain, was specified, and a gene sequence corresponding thereto was synthesized. After cleaving pET302/NT-His which is a His-tag expression vector by the restriction enzyme EcoRI, the resulting fragments were treated with alkaline phosphatase as a dephosphorylation treatment, and then mixed with the above-synthesized gene sequence, and a ligation reaction was performed with using DNA Ligation Kit Ver. 2 (Takara Bio, Inc.).

A recombinant VZVgE plasmid integrated with a target gene was transfected into a recombinant protein expression host *E. coli* BL(DE3)pLysS (Novagen). A transformant was cultured on an LB agar plate culture medium, and an obtained colony was cultured in an LB liquid culture medium. Further, 1 mM IPTG (Takara Bio, Inc.) was added thereto to induce the expression of the recombinant VZVgE, and then, the *E. coli* was recovered. The recovered E. coli was resuspended in a lysate buffer [0.5% Triton X-100 (Sigma), 10 mM imidazole, 20 mM phosphate, and 0.5 M NaCl (pH 7.4) (Amersham)], and lysed by a sonication treatment, and then, the recombinant VZVgE was purified using His trap Kit (Amersham). The purified protein was dialyzed against phosphate buffered saline (hereinafter, referred to as "PBS"), and thus, the target recombinant VZVgE was prepared.

By using the obtained recombinant VZVgE as the immunization antigen, a monoclonal antibody for the recombinant VZVgE (hereinafter referred to as "anti-VZVgE antibody") was prepared. The preparation of the monoclonal antibody was performed according to a conventional method as follows. After mixing 100 µg of the recombinant VZVgE with the same amount of Adjuvant Complete Freund (Difco), a mouse (BALB/c, 5 weeks of age, Japan SLC, Inc.) was immunized with the mixture 3 times, and then, the spleen cells thereof were used for cell fusion. For the cell fusion, Sp2/0-Agl4 cells (Shulman et al., Nature, 276, 269-270, 1978) which were mouse myeloma cells were used. In the culturing of the cells, a culture medium obtained by adding 0.3 mg/mL L-glutamine, 100 units/mL penicillin G potassium, 100 µg/mL streptomycin sulfate, and 40 µg/mL Gentacin to Dulbecco's modified Eagle medium (Gibco) (DMEM), and further adding fetal bovine serum (JRH) thereto to give a final concentration of 10% was used. The cell fusion was performed by mixing the spleen cells of the immunized mouse with the Sp2/0-Agl4 cells, and adding a polyethylene glycol solution (Sigma) thereto. The resulting fused cells were cultured in HAT-DMEM [serum-supplemented DMEM containing 0.1 mM sodium hypoxanthine, 0.4 µM aminopterin, and 0.016 mM thymidine (Gibco)], and the production of the antibody in the culture supernatant was confirmed by the enzyme-linked immunoassay (ELISA). The cells which were positive for the production of the antibody were cultured in HT-DMEM [serum-supplemented DMEM containing 0.1 mM sodium hypoxanthine and 0.16 mM thymidine], and further, the culturing was continued in serum-supplemented DMEM.

The cloned cells were inoculated into the abdominal cavity of a mouse (BALB/c, Retire, Japan SLC, Inc.) into which 2,6,10,14-tetramethylpentadecane (Sigma) was inoculated in advance, and then, the ascites thereof was collected. The ascites was applied to a protein G column, whereby the monoclonal antibody was purified.

Finally, 23 clones of cells which produce a monoclonal antibody which recognizes VZVgE were obtained.

### [Test Example 2] Screening of Cell which Produces Antibody that Recognizes Amino Acid Sequence at Positions 1 to 188 of VZVgE

In order to screen a cell which produces an antibody that recognizes the amino acid sequence at positions 1 to 188 of VZVgE from the 23 clones of cells which produce a monoclonal antibody that recognizes VZVgE obtained in Test Example 1, the following experiment was performed.

In the same manner as in Test Example 1, a recombinant protein composed of the amino acid sequence at positions 1 to 188 of VZVgE composed of the amino acid sequence of SEQ ID NO: 1 was prepared and used in the following experiment.

After mixing 0.01 mg/mL solution of the prepared VZVgE recombinant protein (amino acid sequence at positions 1 to 188) with the same amount of 2x Tris-Glycine SDS Sample Buffer (manufactured by TEFCO) supplemented with 10% 2-mercaptoethanol, heating was carried out at 100°C for 10 minutes, and the resulting mixture was subjected to SDS-PAGE. The SDS-PAGE was performed according to a known standard method using Ready Gel J5-20% 12 well (manufactured by Bio-Rad, Inc.). A protein was transferred from the gel after the electrophoresis to Sequi-Blot PVDF Membrane (manufactured by Bio-Rad, Inc.) using a blotting device (manufactured by Bio-Rad, Inc.). The PVDF membrane after the transfer was blocked with ImmunoBlock (DS Pharma Laboratories) at room temperature for 1 hour.

The blocking solution was removed, and the PVDF membrane was washed with PBS containing 0.05% Tween 20 (trade name) (hereinafter, referred to as "T-PBS") for 10 minutes three times, then the resulting PVDF membrane was incubated at room temperature for 1 hour together with the culture supernatant containing the monoclonal antibody produced from each of the 23 clones of monoclonal antibody-producing cells obtained in Test Example 1. Each antibody was prepared at a concentration of 10 µg/mL and was allowed to react with the VZVgE recombinant protein in an amount of 1.0 µg per lane. After washing the PVDF membrane with T-PBS for 10 minutes three times, the PVDF membrane was incubated at room temperature for 30 minutes together with alkaline phosphatase-labeled anti-mouse IgG (manufactured by Sigma, Inc.) diluted 5000-fold with T-PBS. After washing with T-PBS for 10 minutes three times, the PVDF membrane was incubated together with 1-Step™ NBT/BCIP (manufactured by Pierce, Inc.) that is a chromogenic substrate, whereby the antibody bound to the PVDF membrane was visualized. As a result, a plurality of bands of 21 kDa (corresponding to the amino acid sequence at positions 1 to 188 of VZVgE) could be detected from the 23 monoclonal antibodies. Two types of antibodies were arbitrarily selected from the antibodies to be subjected to the below-mentioned immunochromatographic analysis test, and the hybridomas which produce the antibody were cloned, then, these two independent clones were selected and named Hybridomas A and B, respectively. Further, the antibodies produced by Hybridomas A and B were named Antibodies A and B, respectively. Each of these antibodies is an antibody which recognizes the amino acid sequence at positions 1 to 188 of VZVgE. The subclasses of the monoclonal antibodies obtained from the two hybridoma strains were both IgG1.

### [Test Example 3] Immunochromatographic Analysis

In this test, an immunochromatographic analysis device using Antibody A or B obtained in Test Example 2 in either one of the labeling substance retaining part and the detection part was prepared, and an immunochromatographic analysis was performed.

### <Preparation of Immunochromatographic Analysis Device>

### (1) Preparation of Sample Addition Part

As a sample addition part, a non-woven fabric composed of glass fiber (manufactured by Millipore, Inc., 300 mm × 30 mm) was used.

### (2) Preparation of Labeling Substance Retaining Part

To 0.5 mL of a colloidal gold suspension (manufactured by Tanaka Kikinzoku Kogyo K.K., LC 40 nm), 0.1 mL of an antibody (either one of Antibodies A and B) diluted with a phosphate buffer solution (pH 7.4) to a concentration of 0.05 mg/mL was added, and the resulting mixture was left to stand at room temperature for 10 minutes.

Subsequently, 0.1 mL of a phosphate buffer solution (pH 7.4) containing 1 % by mass bovine serum albumin (BSA) was added thereto, and the resulting mixture was left to stand at room temperature for an additional 10 minutes. Thereafter, the mixture was thoroughly stirred, and then centrifuged at 8000 × g for 15 minutes. After removing the supernatant, 0.1 mL of a phosphate buffer solution (pH 7.4) containing 1 % by mass BSA was added thereto. According to the above-mentioned procedure, a labeling substance solution was prepared.

A solution obtained by adding 300 µL of a 10 % by mass trehalose aqueous solution and 1.8 mL of distilled water to 300 µL of the above-prepared labeling substance solution was added uniformly to a 12 mm × 300 mm glass fiber pad (manufactured by Millipore, Inc.), followed by drying with a vacuum dryer, whereby a labeling substance retaining part was prepared.

### (3) Preparation of Chromatographic Medium Part and Detection Part

As a membrane, a sheet composed of nitrocellulose (manufactured by Millipore, Inc., trade name: HF 120, 300 mm × 25 mm) was used.

Subsequently, 150 µL of a solution obtained by diluting an antibody (either one of Antibodies A and B) with a phosphate buffer solution (pH 7.4) containing 5 % by mass isopropyl alcohol to a concentration of 1.0 mg/mL was applied to a detection region (detection line) on the dried membrane in a line with a width of 1 mm in an amount of 1 µL/mm (25 µL per sheet) with using a dispenser for immunochromatography "XYZ 3050" (manufactured by BioDot, Inc.).

Further, in order to confirm the presence or absence of development of a gold nanoparticle labeling reagent or the developing speed, on the downstream of the detection region, a solution obtained by diluting a goat-derived antiserum having affinity in a wide range for the gold nanoparticle labeling substance with a phosphate buffer solution (pH 7.4) was applied to a control region. Thereafter, the solution was dried at 50°C for 30 minutes, and then dried overnight at room temperature, whereby a chromatographic medium part and a detection part were prepared.

### (4) Preparation of Immunochromatographic Analysis Device

Subsequently, to a base material composed of a backing sheet, the sample addition part, the labeling substance retaining part, the chromatographic medium part having the detection part, and a non-woven cloth made of glass fiber as an absorption part for absorbing the developed sample and labeling substance were sequentially bonded. Then, the resulting material was cut to a width of 5 mm by a cutting machine, whereby an immunochromatographic analysis device was prepared. The length of the labeling substance retaining part in the sample development direction was set to 12 mm.

### (5) Specimen Diluent

A 50 mM HEPES buffer solution (pH 7.5) containing a mixture of 1 % by mass of a nonionic surfactant NP-40 (manufactured by Nacalai Tesque, Inc., trade name: Nonidet P-40, HLB value: 17.7) and Nonion MN-811 (manufactured by NOF CORPORATION, trade name: Nonion MN-811, HLB value: 8.3) at a mass ratio of 1:1 was prepared and used as a specimen diluent for diluting a specimen.

### <Measurement>

The color development intensity in the detection part was measured in a case where the immunochromatographic analysis device prepared above with using 0.1 mg/mL of the VZVgE (amino acid sequence at positions 1 to 188) recombinant protein prepared in Test Example 2 as the antigen was used. The antigen was diluted 100-fold with the above-prepared specimen diluent, and the resulting solution was used as solution containing specimen.

Each of the above-prepared specimen-containing solutions in an amount of 150 µL was placed on the sample addition part of the immunochromatographic analysis device and developed, and the degree of color development (color development intensity) in the detection part was confirmed by visual observation. The results are shown in Table 1.

In this connection, the evaluation criteria in Table 1 are as follows.
±: Color development can be confirmed, but the color is very faint.
++: Strong color development can be confirmed.

### [Table 1]

**Table 1**

| | | On the detection part side | |
|---|---|---|---|
| | | Antibody A | Antibody B |
| On the labeling substance retaining part side | Antibody A | ± | ++ |
| | Antibody B | ++ | ± |

From the results shown in Table 1, it was found that by using an antibody which recognizes the amino acid sequence at positions 1 to 188 of VZVgE in the immunochromatographic analysis device, VZV can be detected.

### [Test Example 4] Screening of Antibody which Recognizes Amino Acid Sequence at Positions 48 to 135 of VZVgE

In order to screen an antibody which recognizes the amino acid sequence at positions 48 to 135 of VZVgE among the monoclonal antibodies for VZVgE obtained in Test Example 1, the following experiment was performed.

A 0.01 mg/mL VZVgE recombinant protein solution (CalBioreagents, Inc.) was mixed with the same amount of 2x Tris-Glycine SDS Sample Buffer (manufactured by TEFCO) supplemented with 10% 2-mercaptoethanol, followed by heating at 100°C for 10 minutes, and the resulting mixture was subjected to SDS-PAGE. The SDS-PAGE was performed according to a known standard method using Ready Gel J5-20% 12 well (manufactured by Bio-Rad, Inc.). A protein was transferred from the gel after the electrophoresis to Sequi-Blot PVDF Membrane (manufactured by Bio-Rad, Inc.) with using a blotting device (manufactured by Bio-Rad, Inc.). The PVDF membrane after the transfer was blocked with ImmunoBlock (DS Pharma Laboratories) at room temperature for 1 hour.

The blocking solution was removed, and the PVDF membrane was washed with PBS containing 0.05% Tween 20 (trade name) (hereinafter, referred to as "T-PBS") for 10 minutes three times, then the resulting PVDF membrane was incubated at room temperature for 1 hour together with the culture supernatant containing the monoclonal antibody produced from each of the 23 clones of monoclonal antibody-producing cells obtained in Test Example 1. Each antibody was prepared at a concentration of 10 µg/mL and was allowed to react with the VZVgE recombinant protein in an amount of 1.0 µg per lane. After washing the PVDF membrane with T-PBS for 10 minutes three times, the PVDF membrane was incubated at room temperature for 30 minutes together with alkaline phosphatase-labeled anti-mouse IgG (manufactured by Sigma, Inc.) diluted 5000-fold with T-PBS. After washing with T-PBS for 10 minutes three times, the PVDF membrane was incubated together with 1-Step™ NBT/BCIP (manufactured by Pierce, Inc.) which is a chromogenic substrate, whereby the antibody bound to the PVDF membrane was visualized. As a result, six bands of 11 kDa (corresponding to the amino acid sequence at positions 48 to 135 of VZVgE) corresponding to the VZVgE recombinant protein could be detected from the 23 monoclonal antibodies. These six independent clones were selected by cloning the hybridomas which produce the antibodies and named Hybridomas 1, 2, 3, 4, 5, and 6, respectively. Further, the antibodies produced by Hybridomas 1, 2, 3, 4, 5, and 6 were named Antibodies 1, 2, 3, 4, 5, and 6, respectively. In this connection, Antibody 2 corresponds to Antibody A in Test Examples 2 and 3, and Antibody 6 corresponds to Antibody B in Test Examples 2 and 3. The subclasses of the monoclonal antibodies obtained from the six hybridoma strains were all IgG1.

### [Test Example 5] Screening of Antibodies which Recognize Amino Acid Sequences at Positions 48 to 88, at Positions 89 to 107, and at Positions 108 to 135 of VZVgE

VZVgE peptide fragments (amino acid sequences at positions 48 to 88, at positions 89 to 107, and at positions 108 to 135 of SEQ ID NO: 1) at 0.01 mg/mL were synthesized by a peptide solid-phase synthesis method which is a conventional peptide chemical synthesis method, and the following experiment was performed. Each of the above-prepared peptide fragments was mixed with the same amount of 2x Tris-Glycine SDS Sample Buffer (manufactured by TEFCO) supplemented with 10% 2-mercaptoethanol, followed by heating at 100°C for 10 minutes, and the resulting mixture was subjected to SDS-PAGE. The SDS-PAGE was performed according to a known standard method with using Ready Gel J5-20% 12 well (manufactured by Bio-Rad, Inc.). A protein was transferred from the gel after the electrophoresis to Sequi-Blot PVDF Membrane (manufactured by Bio-Rad, Inc.) with using a blotting device (manufactured by Bio-Rad, Inc.). The PVDF membrane after the transfer was blocked with ImmunoBlock (DS Pharma Laboratories) at room temperature for 1 hour. The blocking solution was removed, and the PVDF membrane was washed with PBS containing 0.05% Tween 20 (trade name) (hereinafter, referred to as "T-PBS") for 10 minutes three times, then the resulting PVDF membrane was incubated at room temperature for 1 hour together with the above-prepared Antibody 1, 2, 3, 4, 5, or 6. Each antibody was prepared at a concentration of 10 µg/mL and was allowed to react with the VZVgE recombinant protein in an amount of 1.0 µg per lane. After washing the PVDF membrane with T-PBS for 10 minutes three times, the PVDF membrane was incubated at room temperature for 30 minutes together with alkaline phosphatase-labeled anti-mouse IgG (manufactured by Sigma, Inc.) diluted 5000-fold with T-PBS. After washing with T-PBS for 10 minutes three times, the PVDF membrane was incubated together with 1-Step™ NBT/BCIP (manufactured by Pierce, Inc.) which is a chromogenic substrate, whereby the antibody bound to the PVDF membrane was visualized. The results are shown in Table 2 in which "-" indicates that a band corresponding to any of the VZVgE peptide fragments (amino acid sequences at positions 48 to 88, at positions 89 to 107, and at positions 108 to 135 of SEQ ID NO: 1) could not be detected, and "+" indicates that the band could be detected.

### [Table 2]

**Table 2**

| | Amino acid seq uence of VZVgE peptide fragment | | |
|---|---|---|---|
| | 48 to 88 | 89 to 107 | 108 to 135 |
| Antibody 1 | + | - | - |
| Antibody 2 | + | - | - |
| Antibody 3 | + | - | - |
| Antibody 4 | - | - | + |
| Antibody 5 | - | - | + |
| Antibody 6 | - | + | - |

As a result, it was found that Antibodies 1 to 3 recognize the amino acid sequence at positions 48 to 88 of VZVgE, Antibodies 4 and 5 recognize the amino acid sequence at positions 108 to 135 of VZVgE, and Antibody 6 recognizes the amino acid sequence at positions 89 to 107 of VZVgE.

### [Test Example 6] Immunochromatographic Analysis

In this test, an immunochromatographic analysis device using any of Antibodies 1 to 6 obtained in Test Example 4 was prepared, and an immunochromatographic analysis was performed.

### <Preparation of Immunochromatographic Analysis Device>

### (1) Preparation of Sample Addition Part

As a sample addition part, a non-woven fabric composed of glass fiber (manufactured by Millipore, Inc., 300 mm × 30 mm) was used.

### (2) Preparation of Labeling Substance Retaining Part

To 0.5 mL of a colloidal gold suspension (manufactured by Tanaka Kikinzoku Kogyo K.K., LC 40 nm), 0.1 mL of an antibody (any one of Antibodies 1 to 6) diluted with a phosphate buffer solution (pH 7.4) to a concentration of 0.05 mg/mL was added, and the resulting mixture was left to stand at room temperature for 10 minutes.

Subsequently, 0.1 mL of a phosphate buffer solution (pH 7.4) containing 1 % by mass bovine serum albumin (BSA) was added thereto, and the resulting mixture was left to stand at room temperature for an additional 10 minutes. Thereafter, the mixture was thoroughly stirred, and then centrifuged at 8000 × g for 15 minutes. After removing the supernatant, 0.1 mL of a phosphate buffer solution (pH 7.4) containing 1 % by mass BSA was added thereto. According to the above-mentioned procedure, a labeling substance solution was prepared.

A solution obtained by adding 300 µL of a 10 % by mass trehalose aqueous solution and 1.8 mL of distilled water to 300 µL of the above-prepared labeling substance solution was added uniformly to a 12 mm × 300 mm glass fiber pad (manufactured by Millipore, Inc.), followed by drying with a vacuum dryer, whereby a labeling substance retaining part was prepared.

### (3) Preparation of Chromatographic Medium Part and Detection Part

As a membrane, a sheet composed of nitrocellulose (manufactured by Millipore, Inc., trade name: HF 120, 300 mm × 25 mm) was used.

Subsequently, 150 µL of a solution obtained by diluting an antibody (any one of Antibodies 1 to 6) with a phosphate buffer solution (pH 7.4) containing 5 % by mass isopropyl alcohol to a concentration of 1.0 mg/mL was applied to a detection region (detection line) on the dried membrane in a line with a width of 1 mm in an amount of 1 µL/mm (25 µL per sheet) using a dispenser for immunochromatography "XYZ 3050" (manufactured by BioDot, Inc.).

Further, in order to confirm the presence or absence of development of a gold nanoparticle labeling reagent or the developing speed, on the downstream of the detection region, a solution obtained by diluting a goat-derived antiserum having affinity in a wide range for the gold nanoparticle labeling substance with a phosphate buffer solution (pH 7.4) was applied to a control region. Thereafter, the solution was dried at 50°C for 30 minutes, and then dried overnight at room temperature, whereby a chromatographic medium part and a detection part were prepared.

### (4) Preparation of Immunochromatographic Analysis Device

Subsequently, to a base material composed of a backing sheet, the sample addition part, the labeling substance retaining part, the chromatographic medium part having the detection part, and a non-woven cloth made of glass fiber as an absorption part for absorbing the developed sample and labeling substance were sequentially bonded. Then, the resulting material was cut to a width of 5 mm by a cutting machine, whereby an immunochromatographic analysis device was prepared. The length of the labeling substance retaining part in the sample development direction was set to 12 mm.

### (5) Specimen Diluent

A 50 mM HEPES buffer solution (pH 7.5) containing a mixture of 1 % by mass of a nonionic surfactant NP-40 (manufactured by Nacalai Tesque, Inc., trade name: Nonidet P-40, HLB value: 17.7) and Nonion MN-811 (manufactured by NOF CORPORATION, trade name: Nonion MN-811, HLB value: 8.3) at a mass ratio of 1:1 was prepared and used as a specimen diluent for diluting a specimen.

### <Measurement>

The color development intensity in the detection part was measured in a case where the immunochromatographic analysis device prepared above using 0.01 mg/mL of the VZVgE recombinant protein (CalBioreagents, Inc.) as the antigen was used. The antigen was diluted 100-fold with the above-prepared specimen diluent, and the resulting solution was used as solution containing specimen.

Each of the above-prepared specimen-containing solutions in an amount of 150 µL was added onto the sample addition part of the immunochromatographic analysis device and developed, and the degree of color development (color development intensity) in the detection part was confirmed by visual observation. The results are shown in Table 3.

In this connection, the evaluation criteria in Table 3 are as follows.
-: Color development cannot be confirmed.
±: Although color development can be confirmed, the color is very faint.
+: Color development can be confirmed.
++: Strong color development can be confirmed.
+++: Very strong color development can be confirmed.

### [Table 3]

**Table 3**

| | | On the detection part side | | | | | |
|---|---|---|---|---|---|---|---|
| | | No. 1 | No. 2 | No. 3 | No. 4 | No. 5 | No. 6 |
| On the labeling substance retaining part side | No. 1 | - | ± | ± | +++ | +++ | + |
| | No. 2 | ± | - | ± | +++ | +++ | ++ |
| | No. 3 | ± | ± | - | ++ | +++ | + |
| | No. 4 | + | +++ | ++ | - | ± | + |
| | No. 5 | + | +++ | ++ | ± | - | + |
| | No. 6 | ± | + | ± | + | + | - |

From the results shown in Table 3, it was found that by using an antibody which recognizes the amino acid sequence at positions 48-135 of VZVgE in the immunochromatographic analysis device, VZV can be detected. Particularly, in a case where an antibody (Antibody 4 or 5) which recognizes the amino acid sequence at positions 108 to 135 of VZVgE is incorporated in at least one of the labeling substance retaining part and the detection part, VZV can be more strongly detected.

### [Test Example 7] Immunochromatographic Analysis Using Actual Specimen

Test Example 6 was repeated except that as the specimen containing VZV, a blister's fluid of a person infected with VZV was used. The blister's fluid was collected by puncturing a blister of a test subject who is a person infected with VZV. Further, the collected blister's fluid was diluted 10-fold with the above-prepared specimen diluent, and the resulting solution was used as solution containing specimen.

Each of the above-prepared specimen-containing solutions in an amount of 150 µL was added onto the sample addition part of the immunochromatographic analysis device and developed, and the degree of color development (color development intensity) in the detection part was confirmed by visual observation. The results are shown in Table 4.

In this connection, the evaluation criteria in Table 4 are as follows.
-: Color development cannot be confirmed.
±: Although color development can be confirmed, the color is very faint.
+: Color development can be confirmed.
++: Strong color development can be confirmed.
+++: Very strong color development can be confirmed.

### [Table 4]

**Table 4**

| | | On the detection part side | | | | | |
|---|---|---|---|---|---|---|---|
| | | No. 1 | No. 2 | No. 3 | No. 4 | No. 5 | No. 6 |
| On the labeling substance retaining part side | No. 1 | - | ± | ± | +++ | +++ | + |
| | No. 2 | + | - | ± | +++ | +++ | ++ |
| | No. 3 | ± | ± | - | ++ | +++ | + |
| | No. 4 | + | +++ | ++ | - | ± | + |
| | No. 5 | + | +++ | ++ | ± | - | + |
| | No. 6 | ± | + | ± | + | + | - |

Also in a case where the specimen was replaced with an actual specimen, the same results as in Test Example 6 in which the VZVgE recombinant protein was used as an antigen were obtained.

The present application is based on Japanese Patent Application (Japanese Patent Application No. 2015-230928) filed on November 26, 2015.

### Reference Signs List

1. sample addition part
2. labeling substance retaining part
3. chromatographic medium part
4. detection part
5. absorption part
6. backing sheet

## Claims

1. An immunochromatographic analysis device, which is an immunochromatographic analysis device for detecting Varicella-Zoster Virus (VZV), comprising a sample addition part, a labeling substance retaining part, a chromatographic medium part having a detection part, and an absorption part,
wherein at least one of the labeling substance retaining part and the detection part comprises an antibody which recognizes the amino acid sequence at positions 1 to 188 of glycoprotein E of the Varicella-Zoster Virus (VZVgE) composed of the amino acid sequence of SEQ ID NO: 1.

2. An immunochromatographic analysis device, which is an immunochromatographic analysis device for detecting Varicella-Zoster Virus (VZV), comprising a sample addition part, a labeling substance retaining part, a chromatographic medium part having a detection part, and an absorption part,
wherein at least one of the labeling substance retaining part and the detection part comprises an antibody which recognizes the amino acid sequence at positions 108 to 135 of glycoprotein E of the Varicella-Zoster Virus (VZVgE) composed of the amino acid sequence of SEQ ID NO: 1.

3. The immunochromatographic analysis device according to claim 2, wherein at least one of the labeling substance retaining part and the detection part comprises an antibody which recognizes the amino acid sequence at positions 108 to 135 of glycoprotein E of the varicella-zoster virus (VZV gE) composed of the amino acid sequence of SEQ ID NO: 1, and the other part comprises an antibody which recognizes the amino acid sequence at positions 48 to 88 of glycoprotein E of the varicella-zoster virus (VZV gE) composed of the amino acid sequence of SEQ ID NO: 1.

4. The immunochromatographic analysis device according to any one of claims 1 to 3, wherein a sample to be added to the sample addition part is a blister's fluid containing the Varicella-Zoster Virus.

5. The immunochromatographic analysis device according to any one of claims 1 to 4, wherein a labeling substance contained in the labeling substance retaining part is a gold nanoparticle, and the labeling substance retaining part comprises the gold nanoparticles in an amount of 0.05 to 1.5 µg/cm².

6. An immunochromatographic analysis kit comprising the immunochromatographic analysis device according to any one of claims 1 to 5, and a specimen diluent for diluting and developing a specimen.

7. The immunochromatographic analysis kit according to claim 6, wherein the specimen diluent contains at least one type of nonionic surfactant.

8. An immunochromatographic analysis method, which is a method for detecting Varicella-Zoster Virus (VZV) in a specimen using an immunochromatographic analysis device including a sample addition part, a labeling substance retaining part, a chromatographic medium part having a detection part, and an absorption part, wherein at least one of the labeling substance retaining part and the detection part of the immunochromatographic analysis device contains an antibody which recognizes the amino acid sequence at positions 1 to 188 of glycoprotein E of the Varicella-Zoster Virus (VZVgE) composed of the amino acid sequence of SEQ ID NO: 1, and the method includes the following steps (1) to (4):
(1) a step of adding solution containing specimen obtained by diluting a specimen with a specimen diluent to the sample addition part;
(2) a step of allowing an antibody retained in the labeling substance retaining part to recognize Varicella-Zoster Virus;
(3) a step of developing the specimen and the antibody in the chromatographic medium part as a mobile phase; and
(4) a step of detecting Varicella-Zoster Virus in the developed mobile phase by an antibody contained in the detection part.

## Patentansprüche

1. Immunchromatographische Analysevorrichtung, die eine immunchromatographische Analysevorrichtung zur Detektion von Varicella-Zoster-Virus (VZV) ist, umfassend einen Probenhinzugabeteil, einen Markierungssubstanz haltenden Teil, einen chromatographisches-Medium-Teil mit einem Detektionsteil und einen Absorptionsteil,
wobei wenigstens einer von dem Markierungssubstanz haltenden Teil und dem Detektionsteil einen Antikörper umfasst, der die Aminosäuresequenz an den Positionen 1 bis 188 von Glycoprotein E des Varicella-Zoster-Virus (VZVgE), gebildet aus der Aminosäuresequenz von SEQ ID NO: 1, erkennt.

2. Immunchromatographische Analysevorrichtung, die eine immunchromatographische Analysevorrichtung zur Detektion von Varicella-Zoster-Virus (VZV) ist, umfassend einen Probenhinzugabeteil, einen Markierungssubstanz haltenden Teil, einen chromatographisches-Medium-Teil mit einem Detektionsteil und einen Absorptionsteil,
wobei wenigstens einer von dem Markierungssubstanz haltenden Teil und dem Detektionsteil einen Antikörper umfasst, der die Aminosäuresequenz an den Positionen 108 bis 135 von Glycoprotein E des Varicella-Zoster-Virus (VZVgE), gebildet aus der Aminosäuresequenz von SEQ ID NO: 1, erkennt.

3. Immunchromatographische Analysevorrichtung gemäß Anspruch 2, wobei wenigstens einer von dem Markierungssubstanz haltenden Teil und dem Detektionsteil einen Antikörper umfasst, der die Aminosäuresequenz an den Positionen 108 bis 135 von Glycoprotein E des Varicella-Zoster-Virus (VZV gE), gebildet aus der Aminosäuresequenz von SEQ ID NO: 1, erkennt und der andere Teil einen Antikörper umfasst, der die Aminosäuresequenz an den Positionen 48 bis 88 von Glycoprotein E des Varicella-Zoster-Virus (VZV gE), gebildet aus der Aminosäuresequenz von SEQ ID NO: 1, erkennt.

4. Immunchromatographische Analysevorrichtung gemäß irgendeinem der Ansprüche 1 bis 3, wobei eine zu dem Probenhinzugabeteil zuzugebende Probe ein das Varicella-Zoster-Virus enthaltendes Blasenfluid ist.

5. Immunchromatographische Analysevorrichtung gemäß irgendeinem der Ansprüche 1 bis 4, wobei eine in dem Markierungssubstanz haltenden Teil enthaltene Markierungssubstanz ein Goldnanopartikel ist und der Markierungssubstanz haltende Teil die Goldnanopartikel in einer Menge von 0,05 bis 1,5 µg/cm² umfasst.

6. Immunchromatographisches Analysekit, umfassend die immunchromatographische Analysevorrichtung gemäß irgendeinem der Ansprüche 1 bis 5 und ein Probenverdünnungsmittel zum Verdünnen und Entwickeln einer Probe.

7. Immunchromatographisches Analysekit gemäß Anspruch 6, wobei das Probenverdünnungsmittel wenigstens einen Typ eines nichtionischen Tensids enthält.

8. Immunchromatographisches Analyseverfahren, welches ein Verfahren zur Detektion von Varicella-Zoster-Virus (VZV) in einer Probe ist, unter Verwendung einer immunchromatographischen Analysevorrichtung, die einen Probenhinzugabeteil, einen Markierungssubstanz haltenden Teil, einen chromatographisches-Medium-Teil mit einem Detektionsteil und einen Absorptionsteil einschließt, wobei wenigstens einer von dem Markierungssubstanz haltenden Teil und dem Detektionsteil der immunchromatographischen Analysevorrichtung einen Antikörper enthält, der die Aminosäuresequenz an den Positionen 1 bis 188 von Glycoprotein E des Varicella-Zoster-Virus (VZVgE), gebildet aus der Aminosäuresequenz von SEQ ID NO: 1, erkennt und das Verfahren die folgenden Schritte (1) bis (4) einschließt:
(1) einen Schritt des Hinzugebens von Probe enthaltender Lösung, erhalten durch Verdünnen einer Probe mit einem Probenverdünnungsmittel, zu dem Probenhinzugabeteil;
(2) einen Schritt, bei dem es einem in dem Markierungssubstanz haltenden Teil gehaltenen Antikörper erlaubt wird, Varicella-Zoster-Virus zu erkennen;
(3) einen Schritt des Entwickelns der Probe und des Antikörpers in dem chromatographisches-Medium-Teil als eine mobile Phase; und
(4) einen Schritt des Detektierens von Varicella-Zoster-Virus in der entwickelten mobilen Phase durch einen in dem Detektionsteil enthaltenen Antikörper.

## Revendications

1. Dispositif d'analyse immunochromatographique, qui est un dispositif d'analyse immunochromatographique pour détecter le virus varicelle-zona (VZV), comprenant une partie d'ajout d'échantillon, une partie de stockage de substance de marquage, une partie de milieu de chromatographie présentant une partie de détection, et une partie d'absorption,
dans lequel au moins l'une parmi la partie de stockage de substance de marquage et la partie de détection comprend un anticorps qui reconnaît la séquence d'acides aminés aux positions 1 à 188 de la glycoprotéine E du virus varicelle-zona (VZVgE) composée de la séquence d'acides aminés de SEQ ID N° : 1.

2. Dispositif d'analyse immunochromatographique, qui est un dispositif d'analyse immunochromatographique pour détecter le virus varicelle-zona (VZV), comprenant une partie d'ajout d'échantillon, une partie de stockage de substance de marquage, une partie de milieu de chromatographie présentant une partie de détection, et une partie d'absorption,
dans lequel au moins l'une parmi la partie de stockage de substance de marquage et la partie de détection comprend un anticorps qui reconnaît la séquence d'acides aminés aux positions 108 à 135 de la glycoprotéine E du virus varicelle-zona (VZVgE) composée de la séquence d'acides aminés de SEQ ID N° : 1.

3. Dispositif d'analyse immunochromatographique selon la revendication 2, dans lequel au moins l'une parmi la partie de stockage de substance de marquage et la partie de détection comprend un anticorps qui reconnaît la séquence d'acides aminés aux positions 108 à 135 de la glycoprotéine E du virus varicelle-zona (VZVgE) composée de la séquence d'acides aminés de SEQ ID N° : 1, et l'autre partie comprend un anticorps qui reconnaît la séquence d'acides aminés aux positions 48 à 88 de la glycoprotéine E du virus varicelle-zona (VZVgE) composée de la séquence d'acides aminés de SEQ ID N° : 1.

4. Dispositif d'analyse immunochromatographique selon l'une quelconque des revendications 1 à 3, dans lequel un échantillon à ajouter à la partie d'ajout d'échantillon est un liquide de vésicule contenant le virus varicelle-zona.

5. Dispositif d'analyse immunochromatographique selon l'une quelconque des revendications 1 à 4, dans lequel une substance de marquage contenue dans la partie de stockage de substance de marquage est une nanoparticule d'or, et la partie de stockage de substance de marquage comprend les nanoparticules d'or à raison de 0,05 à 1,5 µg/cm².

6. Kit d'analyse immunochromatographique comprenant le dispositif d'analyse immunochromatographique selon l'une quelconque des revendications 1 à 5, et un diluant d'échantillon pour diluer et développer un échantillon.

7. Kit d'analyse immunochromatographique selon la revendication 6, dans lequel le diluant d'échantillon contient au moins un type de tensioactif non ionique.

8. Procédé d'analyse immunochromatographique, qui est un procédé pour détecter le virus varicelle-zona (VZV) dans un échantillon en utilisant un dispositif d'analyse immunochromatographique incluant une partie d'ajout d'échantillon, une partie de stockage de substance de marquage, une partie de milieu de chromatographie présentant une partie de détection, et une partie d'absorption, dans lequel au moins l'une parmi la partie de stockage de substance de marquage et la partie de détection du dispositif d'analyse immunochromatographique contient un anticorps qui reconnaît la séquence d'acides aminés aux positions 1 à 188 de la glycoprotéine E du virus varicelle-zona (VZVgE) composée de la séquence d'acides aminés de SEQ ID N° : 1, et le procédé inclut les étapes (1) à (4) suivantes :
(1) une étape consistant à ajouter une solution contenant un échantillon obtenue en diluant un échantillon avec un diluant d'échantillon dans la partie d'ajout d'échantillon ;
(2) une étape consistant à permettre à un anticorps stocké dans la partie de stockage de substance de marquage de reconnaître le virus varicelle-zona ;
(3) une étape consistant à développer l'échantillon et l'anticorps dans la partie de milieu de chromatographie comme une phase mobile ; et
(4) une étape consistant à détecter le virus varicelle-zona dans la phase mobile développée par un anticorps contenu dans la partie de détection.


### SEQUENCE LISTING

<110> TANAKA KIKINZOKU KOGYO K.K.
<120> Immunochromatography inspection apparatus for detecting Varicella zoster virus
<130> W521077
<150> JP2015/230928
   <151> 2015-11-26
<160> 2
<170> PatentIn version 3.5
<210> 1
   <211> 623
   <212> PRT
   <213> Varicella zoster virus
<400> 1
   Met Gly Thr Val Asn Lys Pro Val Val Gly Val Leu Met Gly Phe Gly
   1               5                   10                  15
   Ile Ile Thr Gly Thr Leu Arg Ile Thr Asn Pro Val Arg Ala Ser Val
               20                  25                  30
   Leu Arg Tyr Asp Asp Phe His Thr Asp Glu Asp Lys Leu Asp Thr Asn
           35                  40                  45
   Ser Val Tyr Glu Pro Tyr Tyr His Ser Asp His Ala Glu Ser Ser Trp
       50                  55                  60
   Val Asn Arg Gly Glu Ser Ser Arg Lys Ala Tyr Asp His Asn Ser Pro
   65                  70                  75                  80
   Tyr Ile Trp Pro Arg Asn Asp Tyr Asp Gly Phe Leu Glu Asn Ala His
                   85                  90                  95
   Glu His His Gly Val Tyr Asn Gln Gly Arg Gly Ile Asp Ser Gly Glu
               100                 105                 110
   Arg Leu Met Gln Pro Thr Gln Met Ser Ala Gln Glu Asp Leu Gly Asp
           115                 120                 125
   Asp Thr Gly Ile His Val Ile Pro Thr Leu Asn Gly Asp Asp Arg His
       130                 135                 140
   Lys Ile Val Asn Val Asp Gln Arg Gln Tyr Gly Asp Val Phe Lys Gly
   145                 150                 155                 160
   Asp Leu Asn Pro Lys Pro Gln Gly Gln Arg Leu Ile Glu Val Ser Val
                   165                 170                 175
   Glu Glu Asn His Pro Phe Thr Leu Arg Ala Pro Ile Gln Arg Ile Tyr
               180                 185                 190
   Gly Val Arg Tyr Thr Glu Thr Trp Ser Phe Leu Pro Ser Leu Thr Cys
           195                 200                 205
   Thr Gly Asp Ala Ala Pro Ala Ile Gln His Ile Cys Leu Lys His Thr
       210                 215                 220
   Thr Cys Phe Gln Asp Val Val Val Asp Val Asp Cys Ala Glu Asn Thr
   225                 230                 235                 240
   Lys Glu Asp Gln Leu Ala Glu Ile Ser Tyr Arg Phe Gln Gly Lys Lys
                   245                 250                 255
   Glu Ala Asp Gln Pro Trp Ile Val Val Asn Thr Ser Thr Leu Phe Asp
               260                 265                 270
   Glu Leu Glu Leu Asp Pro Pro Glu Ile Glu Pro Gly Val Leu Lys Val
           275                 280                 285
   Leu Arg Thr Glu Lys Gln Tyr Leu Gly Val Tyr Ile Trp Asn Met Arg
       290                 295                 300
   Gly Ser Asp Gly Thr Ser Thr Tyr Ala Thr Phe Leu Val Thr Trp Lys
   305                 310                 315                 320
   Gly Asp Glu Lys Thr Arg Asn Pro Thr Pro Ala Val Thr Pro Gln Pro
                   325                 330                 335
   Arg Gly Ala Glu Phe His Met Trp Asn Tyr His Ser His Val Phe Ser
               340                 345                 350
   Val Gly Asp Thr Phe Ser Leu Ala Met His Leu Gln Tyr Lys Ile His
           355                 360                 365
   Glu Ala Pro Phe Asp Leu Leu Leu Glu Trp Leu Tyr Val Pro Ile Asp
       370                 375                 380
   Pro Thr Cys Gln Pro Met Arg Leu Tyr Ser Thr Cys Leu Tyr His Pro
   385                 390                 395                 400
   Asn Ala Pro Gln Cys Leu Ser His Met Asn Ser Gly Cys Thr Phe Thr
                   405                 410                 415
   Ser Pro His Leu Ala Gln Arg Val Ala Ser Thr Val Tyr Gln Asn Cys
   420 425 430
   Glu His Ala Asp Asn Tyr Thr Ala Tyr Cys Leu Gly Ile Ser His Met
   435 440 445
   Glu Pro Ser Phe Gly Leu Ile Leu His Asp Gly Gly Thr Thr Leu Lys
   450 455 460
   Phe Val Asp Thr Pro Glu Ser Leu Ser Gly Leu Tyr Val Phe Val Val
   465 470 475 480
   Tyr Phe Asn Gly His Val Glu Ala Val Ala Tyr Thr Val Val Ser Thr
   485 490 495
   Val Asp His Phe Val Asn Ala Ile Glu Glu Arg Gly Phe Pro Pro Thr
   500 505 510
   Ala Gly Gln Pro Pro Ala Thr Thr Lys Pro Lys Glu Ile Thr Pro Val
   515 520 525
   Asn Pro Gly Thr Ser Pro Leu Leu Arg Tyr Ala Ala Trp Thr Gly Gly
   530 535 540
   Leu Ala Ala Val Val Leu Leu Cys Leu Val Ile Phe Leu Ile Cys Thr
   545 550 555 560
   Ala Lys Arg Met Arg Val Lys Ala Tyr Arg Val Asp Lys Ser Pro Tyr
   565 570 575
   Asn Gln Ser Met Tyr Tyr Ala Gly Leu Pro Val Asp Asp Phe Glu Asp
   580 585 590
   Ser Glu Ser Thr Asp Thr Glu Glu Glu Phe Gly Asn Ala Ile Gly Gly
   595 600 605
   Ser His Gly Gly Ser Ser Tyr Thr Val Tyr Ile Asp Lys Thr Arg
   610 615 620
<210> 2
   <211> 544
   <212> PRT
   <213> Varicella zoster virus
<400> 2
   Met Gly Thr Val Asn Lys Pro Val Val Gly Val Leu Met Gly Phe Gly
   1 5 10 15
   Ile Ile Thr Gly Thr Leu Arg Ile Thr Asn Pro Val Arg Ala Ser Val
               20                  25                  30
   Leu Arg Tyr Asp Asp Phe His Thr Asp Glu Asp Lys Leu Asp Thr Asn
           35                  40                  45
   Ser Val Tyr Glu Pro Tyr Tyr His Ser Asp His Ala Glu Ser Ser Trp
       50                  55                  60
   Val Asn Arg Gly Glu Ser Ser Arg Lys Ala Tyr Asp His Asn Ser Pro
   65                  70                  75                  80
   Tyr Ile Trp Pro Arg Asn Asp Tyr Asp Gly Phe Leu Glu Asn Ala His
                   85                  90                  95
   Glu His His Gly Val Tyr Asn Gln Gly Arg Gly Ile Asp Ser Gly Glu
               100                 105                 110
   Arg Leu Met Gln Pro Thr Gln Met Ser Ala Gln Glu Asp Leu Gly Asp
           115                 120                 125
   Asp Thr Gly Ile His Val Ile Pro Thr Leu Asn Gly Asp Asp Arg His
       130                 135                 140
   Lys Ile Val Asn Val Asp Gln Arg Gln Tyr Gly Asp Val Phe Lys Gly
   145                 150                 155                 160
   Asp Leu Asn Pro Lys Pro Gln Gly Gln Arg Leu Ile Glu Val Ser Val
                   165                 170                 175
   Glu Glu Asn His Pro Phe Thr Leu Arg Ala Pro Ile Gln Arg Ile Tyr
               180                 185                 190
   Gly Val Arg Tyr Thr Glu Thr Trp Ser Phe Leu Pro Ser Leu Thr Cys
           195                 200                 205
   Thr Gly Asp Ala Ala Pro Ala Ile Gln His Ile Cys Leu Lys His Thr
       210                 215                 220
   Thr Cys Phe Gln Asp Val Val Val Asp Val Asp Cys Ala Glu Asn Thr
   225                 230                 235                 240
   Lys Glu Asp Gln Leu Ala Glu Ile Ser Tyr Arg Phe Gln Gly Lys Lys
                   245                 250                 255
   Glu Ala Asp Gln Pro Trp Ile Val Val Asn Thr Ser Thr Leu Phe Asp
               260                 265                 270
   Glu Leu Glu Leu Asp Pro Pro Glu Ile Glu Pro Gly Val Leu Lys Val
           275                 280                 285
   Leu Arg Thr Glu Lys Gln Tyr Leu Gly Val Tyr Ile Trp Asn Met Arg
       290                 295                 300
   Gly Ser Asp Gly Thr Ser Thr Tyr Ala Thr Phe Leu Val Thr Trp Lys
   305                 310                 315                 320
   Gly Asp Glu Lys Thr Arg Asn Pro Thr Pro Ala Val Thr Pro Gln Pro
                   325                 330                 335
   Arg Gly Ala Glu Phe His Met Trp Asn Tyr His Ser His Val Phe Ser
               340                 345                 350
   Val Gly Asp Thr Phe Ser Leu Ala Met His Leu Gln Tyr Lys Ile His
           355                 360                 365
   Glu Ala Pro Phe Asp Leu Leu Leu Glu Trp Leu Tyr Val Pro Ile Asp
       370                 375                 380
   Pro Thr Cys Gln Pro Met Arg Leu Tyr Ser Thr Cys Leu Tyr His Pro
   385                 390                 395                 400
   Asn Ala Pro Gln Cys Leu Ser His Met Asn Ser Gly Cys Thr Phe Thr
                   405                 410                 415
   Ser Pro His Leu Ala Gln Arg Val Ala Ser Thr Val Tyr Gln Asn Cys
               420                 425                 430
   Glu His Ala Asp Asn Tyr Thr Ala Tyr Cys Leu Gly Ile Ser His Met
           435                 440                 445
   Glu Pro Ser Phe Gly Leu Ile Leu His Asp Gly Gly Thr Thr Leu Lys
       450                 455                 460
   Phe Val Asp Thr Pro Glu Ser Leu Ser Gly Leu Tyr Val Phe Val Val
   465                 470                 475                 480
   Tyr Phe Asn Gly His Val Glu Ala Val Ala Tyr Thr Val Val Ser Thr
                   485                 490                 495
   Val Asp His Phe Val Asn Ala Ile Glu Glu Arg Gly Phe Pro Pro Thr
               500                 505                 510
   Ala Gly Gln Pro Pro Ala Thr Thr Lys Pro Lys Glu Ile Thr Pro Val
                       515                 520                 525
   Asn Pro Gly Thr Ser Pro Leu Leu Arg Tyr Ala Ala Trp Thr Gly Gly
           530                 535                 540